(19) Europäisches Patentamt
European Patent Office
Office européen des brevets

(11) EP 0 750 904 A1

(12) EUROPÄISCHE PATENTANMELDUNG

(43) Veröffentlichungstag:
**02.01.1997 Patentblatt 1997/01**

(51) Int. Cl.[6]: **A61K 7/48**, A61K 7/50

(21) Anmeldenummer: **96101328.1**

(22) Anmeldetag: **31.01.1996**

(84) Benannte Vertragsstaaten:
**DE ES FR GB IT**

(30) Priorität: **08.06.1995 DE 19520859**

(71) Anmelder: **Wella Aktiengesellschaft**
**64274 Darmstadt (DE)**

(72) Erfinder:
- **Bimczok, Rudolf, Dr.**
  **D-64342 Seeheim (DE)**
- **Lang, Günther, Dr.**
  **D-64354 Reinheim (DE)**

# (54) Verwendung einer Betainester und alpha-Hydroxysäuren enthaltenden Zubereitung zur Pflege der Haut

(57) Gegenstand der vorliegenden Anmeldung ist die Verwendung einer Zubereitung mit einem pH-Wert von 1 bis 7 und einem Gehalt an
0,1 bis 20 Gewichtsprozent mindestens eines Betainesters der allgemeinen Formel I

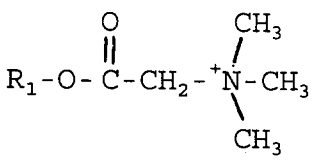 $A^-$ (I),

wobei $R_1$ einen $C_{10}$- bis $C_{30}$-Alkylrest und $A^-$ ein Anion, vorzugsweise ein Halogenidanion, Sulfat-, Methosulfat- oder Phosphatanion darstellt und
0,05 bis 20 Gewichtsprozent mindestens einer α-Hydroxysäure, die ausgewählt ist aus Zitronensäure oder Milchsäure, zur Pflege der Haut
sowie ein neues Hautpflegemittel mit einem pH-Wert von 1 bis 7 und einem Gehalt an einer Kombination aus

a) 0,1 bis 20 Gewichtsprozent mindestens eines Betainesters der allgemeinen Formel (I)
und
b) 0,05 bis 20 Gewichtsprozent mindestens einer α-Hydroxysäure mit Ausnahme von Milchsäure und Zitronensäure.

Sowohl die erfindungsgemäß verwendete Zubereitung als auch das erfindungsgemäße Hautpflegemittel sind sehr gut hautverträglich, die Haut wird hydratisiert und ein angenehmes gepflegtes Hautgefühl wird erzielt.

EP 0 750 904 A1

## Beschreibung

Gegenstand der vorliegenden Erfindung ist die Verwendung einer Betainester und bestimmte α-Hydroxysäuren enthaltenden Zubereitung zur Pflege der Haut sowie neue Hautpflegemittel.

Die menschliche Haut wird durch Einwirkungen verschiedener Art in ihren Eigenschaften negativ beeinflußt. So wird sie durch Umwelteinflüsse, wie zum Beispiel Trockenheit und Kälte, oder durch häufigen Kontakt mit seifen- oder tensidhaltigen Reinigungsmitteln, strapaziert. Kosmetische Hautpflegemittel sollen die Erhaltung und Wiederherstellung des physiologischen Gleichgewichts der Hautoberfläche dienen, gute Bedingungen für eine ständige Hautregeneration schaffen, gleichzeitig vor äußeren Einflüssen schützen, und die Haut beruhigen.

Hautpflegemittel, die üblicherweise in Form von Hautcremes oder Hautlotionen vorliegen, müssen ausgezeichnet hautverträglich sein, sich gut auf der Haut verteilen lassen und rasch einziehen ohne einen störenden Fettglanz auf der Hautoberfläche zu hinterlassen. Die mit Hautpflegemitteln behandelte Haut sollte sich nach der Behandlung glatt und geschmeidig anfühlen.

Darüberhinaus sollte ein Hautpflegemittel eine anhaltende Wirkung entfalten, indem die enthaltenen Wirkstoffe in die Epidermis eindringen, eine Regeneration und Hydratisierung der Hornschichten bewirken und so zur Glättung von Hautfältchen führen.

Obwohl eine Vielzahl von Hautpflegemitteln auf der Basis unterschiedlichster Wirkstoffe bekannt sind, ist es bisher nicht gelungen, ein Hautpflegemittel zur Verfügung zustellen, das in allen Eigenschaften - insbesondere aber bezüglich seiner hydratisierenden Wirkung auf die Hornschicht, seiner Fältchen glättenden Wirkung, seiner Hautverträglichkeit sowie der Vermittlung eines glatten und geschmeidigen Hautgefühls- völlig zufriedenstellend ist.

Es bestand daher die Aufgabe, ein Hautpflegemittel zur Verfügung zu stellen, das die Haut hydratisiert, Fältchen glättet, ein angenehmes Hautgefühl erzeugt und darüber hinaus ausgezeichnet hautverträglich ist.

Es wurde nun gefunden, daß die Verwendung einer Zubereitung mit einem pH-Wert von 1 bis 7, die 0,1 bis 20 Gewichtsprozent, vorzugsweise 0,5 bis 5 Gewichtsprozent, mindestens eines Betainesters der allgemeinen Formel I

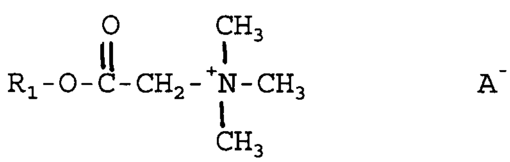

(I),

wobei $R_1$ einen $C_{10}$- bis $C_{30}$-Alkylrest und $A^-$ ein Anion, bevorzugt ein Halogenidanion, beispielsweise ein Bromid- oder Chloridanion, ein Sulfat-, Methosulfat- oder Phosphatanion darstellt und 0,05 bis 20 Gewichtsprozent mindestens einer α-Hydroxysäure, die ausgewählt ist aus Zitronensäure oder Milchsäure, enthält, zur Hautpflege die gestellte Aufgabe löst.

Die Betainester der $C_{10}$- bis $C_{30}$-Fettalkohole der allgemeinen Formel (I) können nach der aus der offengelegten japanischen Patentanmeldung 157 750 von 1983 bekannten Synthese hergestellt werden.

Die Synthese kann durch direkte Veresterung von Fettalkoholen mit dem bei der Zuckerfabrikation als Nebenprodukt anfallenden Betain gemäß dem nachfolgenden Reaktionsschema erfolgen:

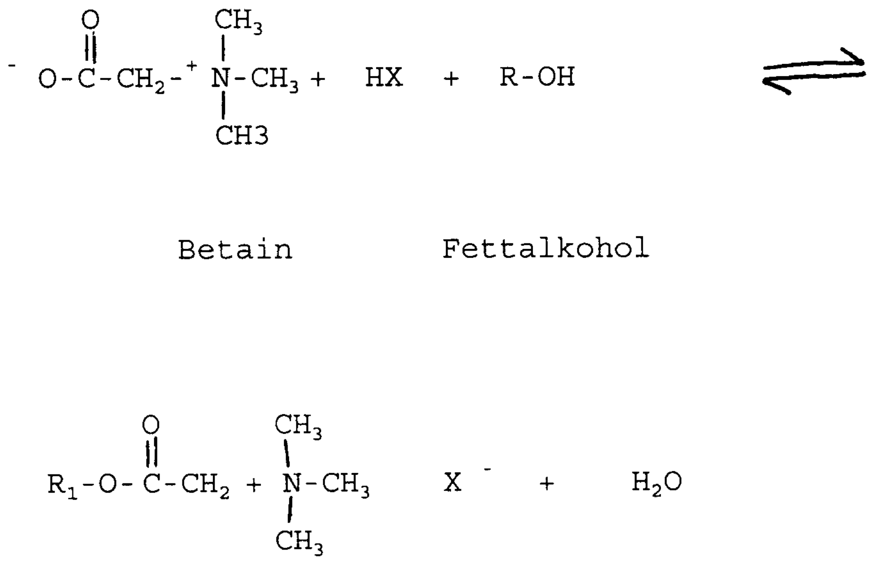

Betain Fettalkohol

Die Betainester der allgemeinen Formel (I) sind darüber hinaus aus der DE-OS 35 27 974 als Bestandteile saurer Haarpflegemittel bekannt.

Im schwach sauren, neutralen oder alkalischen pH-Bereich zerfallen die Betainester in die Bestandteile Fettalkohol und Betain, während sie im sauren Bereich stabil sind. Betain wirkt entzündungshemmend und anti-irritierend auf die Haut. Die Betainester der allgemeinen Formel (I) entfalten bei ihrer erfindungsgemäßen Verwendung eine hautglättende Wirkung, während sie nach der hydrolytischen Spaltung zu Betain und Fettalkohol antirritierend auf die Haut wirken.

Die erfindungsgemäß verwendete Zubereitung ist trotz ihres Gehaltes an $\alpha$-Hydroxysäuren, welche insbesondere bei empfindlicher Haut Hautreizungen hervorrufen können, sehr gut hautverträglich. Die Betainester der allgemeinen Formel (I) wirken daher nicht nur antiirritierend und entzündungshemmend, sondern verbessern die Hautverträglichkeit von $\alpha$-Hydroxysäuren.

Gegenstand der vorliegenden Erfindung ist weiterhin ein Hautpflegemittel mit einem pH-Wert von 1 bis 7, welches eine Kombination aus

a) 0,1 bis 20 Gewichtsprozent, vorzugsweise 0,5 bis 5 Gewichtsprozent, mindestens eines Betainesters der allgemeinen Formel (I)

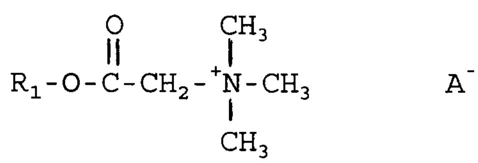 (I),

wobei $R_1$ einen $C_{10}$- bis $C_{30}$-Alkylrest und A- ein Anion, bevorzugt ein Halogenidanion, beispielsweise ein Bromid- oder Chloridanion, ein Sulfat-, Methosulfat- oder Phosphatanion, darstellt und

b) 0,05 bis 20 Gewichtsprozent, vorzugsweise 0,5 bis 6 Gewichtsprozent, mindestens einer $\alpha$-Hydroxysäure mit Ausnahme von Milchsäure und Zitronensäure

enthält.

Das erfindungsgemäße Hautpflegemittel hydratisiert die Haut, glättet Fältchen, erzeugt ein angenehmes gepflegtes Hautgefühl und ist darüber hinaus ausgezeichnet hautverträglich.

Insbesondere die gute Hautverträglichkeit ist überraschend, da $\alpha$-Hydroxysäuren aufgrund ihrer keratolytischen Wirkung Hautreizungen verursachen können.

Das erfindungsgemäße Hautpflegemittel enthält als Komponente b) bevorzugt eine $\alpha$-Hydroxysäure, die ausgewählt ist aus Glykolsäure, Äpfelsäure, Weinsäure, Salicylsäure, Gluconsäure, Glucuronsäure und Zuckersäure.

Sowohl die erfindungsgemäß verwendete Zubereitung als auch das erfindungsgemäße Hautpflegemittel enthalten als Betainester der allgemeinen Formel (I) bevorzugt Betainoctadecylester und/oder Betaindodecylester.

Sowohl die erfindungsgemäß verwendete Zubereitung als auch das erfindungsgemäße Hautpflegemittel können in Form von Lösungen, Suspensionen, Pasten, Gelen oder Emulsionen, vorzugsweise jedoch in Form von Öl-in-Wasser- oder Wasser-in-Öl-Emulsionen, vorliegen und vorzugsweise als Gesichtswasser, Hautcreme, Körperlotion, Gesichtspackung oder Gesichtsmaske konfektioniert sein.

Sowohl die gemäß der vorliegenden Erfindung verwendete Zubereitung als auch das erfindungsgemäße Hautpflegemittel können zusätzlich alle diejenigen Bestandteile enthalten, die in Hautbehandlungsmitteln üblicherweise eingesetzt werden, insbesondere nicht-ionische, kationische, amphotere oder zwitterionische Tenside, beispielsweise oxethylierte Fettalkohole mit 12 bis 18 Kohlenstoffatomen, zum Beispiel mit bis zu 40 Mol Ethylenoxid pro Mol Fettalkohol oxethylierter Lauryl-, Cetyl- oder Stearylalkohol, Alkylbetaine, Alkylaminobetaine, Alkylsulfobetaine und Fettsäurealkylamidobetaine in einer Menge von 0,01 bis 5,0 Gewichtsprozent; Sequestriermittel; Emulgatoren; Naturstoffe, wie zum Beispiel die Vitamine F und B6, D-Panthenol, Aminosäuren wie Betain, Cystein, Alanin, Valin oder Tyrosin oder Pflanzenextrakte, Pigmente, Parfümöle in einer Menge von 0,5 bis 5,0 Gewichtsprozent; Trübungsmittel, wie zum Beispiel Ethylenglykoldistearat, in einer Menge von etwa 0,5 bis 5,0 Gewichtsprozent; Feuchthaltemittel, wie beispielsweise Glycerin, Polyole, Hyaluronsäure und Harnstoff, in einer Menge von 0,05 bis 20 Gewichtsprozent, vorzugsweise 0,1 bis 10 Gewichtsprozent; Perlglanzmittel, wie zum Beispiel ein Gemisch aus Fettsäuremonoalkylolamid und Ethylenglykoldistearat, in einer Menge von etwa 1,0 bis 10,0 Gewichtsprozent; Verdickungsmittel, wie beispielsweise Kokosfettsäurediethanolamid oder Hydroxyalkylcellulose, in einer Menge von 0,1 bis 1,0 Gewichtsprozent; Puffersubstanzen, wie beispielsweise Natriumcitrat oder Natriumphosphat, in einer Menge von 0,1 bis 1,0 Gewichtsprozent; sowie Farbstoffe, wie zum Beispiel Fluorescein-Natriumsalz, Gelb ZN3 (C.I. 47 055), in einer Menge von 0,1 bis 1,0 Gewichtsprozent; weiterhin hautpflegende Zusätze, wie zum Beispiel Fettsäureester, Fettalkohole, Fettsäureglyceride; natürliche, modifizierte natürliche oder synthetische Polymere, wie zum Beispiel kationische, anionische oder nichtionische Cellulosederivate, Chitosan, kationische Chitin- oder Chitosanderivate, Pflegestoffe, wie zum Beispiel Lanolinderivate, Cholesterin, Allantoin, Alpha-Bisabolol, Azulen und Pantothensäure, in einer Menge von 0,1 bis 10 Gewichtsprozent; sowie ferner Lichtschutzmittel; Antioxidantien; Komplexbildner; kosmetische Öle und Wachse sowie Konservierungsstoffe, soweit solche Zusätze nützlich und zweckmäßig erscheinen und mit den Bestandteilen der verwendeten Zubereitung verträglich sind.

Der pH-Wert der gemäß der vorliegenden Erfindung verwendeten Zubereitung als auch des erfindungsgemäßen Hautpflegemittels beträgt vorzugsweise 4 bis 6 und kann, wenn er sich nicht durch die enthaltenen $\alpha$-Hydroxysäuren einstellt, mit physiologisch verträglichen organischen oder anorganischen Säuren oder Basen, beispielsweise mit Benzoesäure, Zitronensäure, Ameisensäure oder Essigsäure, Sorbinsäure, Natriumhydroxid, Ammoniak oder Mono- oder Triethanolamin eingestellt werden. Die erfindungsgemäß verwendete Zubereitung und das erfindungsgemäße Hautpflegemittel können wasserfrei sein oder bis zu 99,5 Gewichtsprozent Wasser enthalten. Bevorzugt weisen die erfindungsgemäß verwendete Zubereitung und das erfindungsgemäße Hautpflegemittel einen Wassergehalt von 50 bis 80 Gewichtsprozent auf.

Die erfindungsgemäß verwendete Zubereitung und das erfindungsgemäße Hautpflegemittel können unter Verwendung eines Treibmittels oder mit Hilfe einer mechanisch betriebenen Sprühvorrichtung oder Schaumerzeugungsvorrichtung versprüht oder als Schaum abgegeben werden.

Wenn das erfindungsgemäße Mittel oder die erfindungsgemäß verwendete Zubereitung mit Hilfe eines Treibmittels versprüht wird, so enthalten sie bevorzugt 3 bis 20 Gewichtsprozent des Treibmittels und werden in einen Druckbehälter abgefüllt.

Als Treibmittel sind beispielsweise niedere Alkane, wie zum Beispiel n-Butan, i-Butan und Propan, oder auch deren Gemische mit Dimethylether sowie ferner bei den in Betracht kommenden Drücken gasförmig vorliegende Treibmittel, wie beispielsweise $N_2$, $N_2O$ und $CO_2$, sowie Gemische der vorstehend genannten Treibmittel geeignet.

Unter mechanischen Sprühvorrichtungen oder Schaumerzeugungsvorrichtungen sind solche Vorrichtungen zu verstehen, welche das Versprühen oder Aufschäumen einer Flüssigkeit ohne Verwendung eines Treibmittels ermöglichen. Als geeignete mechanische Sprühvorrichtung kann beispielsweise eine Sprühpumpe oder ein mit einem Sprühventil versehener elastischer Behälter, in den das erfindungsgemäße kosmetische Mittel unter Druck abgefüllt wird, wobei sich der elastische Behälter ausdehnt und aus dem das Mittel infolge der Kontraktion des elastischen Behälters bei Öffnen des Sprühventils kontinuierlich abgegeben wird, verwendet werden.

Als geeignete mechanische Schaumerzeugungsvorrichtung kann beispielsweise der in der EP-B 0 460 154 beschriebene Aufsatz mit einer Schaumerzeugungsvorrichtung auf einem elastischen Behälter verwendet werden.

Folgende Beispiele sollen den Gegenstand der Erfindung näher erläutern:

## Beispiele

**Beispiel 1:** Hautpflegezubereitung

a)

| | |
|---|---|
| 5,0 g | Betainoctadecylester |
| 3,0 g | Polyglyceryl-3-methylglucosedistearat |
| 3,0 g | Glycerylstearat |
| 20,0 g | Isopropylstearat |
| 1,5 g | Stearylalkohol |
| 5,0 g | Mineralöl |

b)

| | |
|---|---|
| 5,0 g | Glycerin |
| 56,7 g | Wasser |
| 0,3 g | Parfümöl |
| 0,5 g | Zitronensäure |
| 100,0 g | |

Die Hautpflegezubereitung gemäß Beispiel 1 wird hergestellt, in dem die Ölphase a) bei etwa 75 Grad Celsius aufgeschmolzen und unter Rühren in die ebenfalls auf 75 Grad Celsius erwärmte aus Glycerin und Wasser bestehende Wasserphase b) emulgiert wird. Nachdem die Emulsion unter gleichmäßigem Rühren auf 45 Grad Celsius abgekühlt ist, wird das Parfümöl c) eingearbeitet. Der pH-Wert wird mit Zitronensäure auf pH=4 eingestellt.

Die Hautpflegezubereitung gemäß Beispiel 1 ist eine weiche Creme, die rasch in die Haut einzieht, ausgezeichnet hautverträglich ist und die Hautfeuchtigkeit über einen Zeitraum von mehreren Stunden erhöht.

**Beispiel 2:** Hautcreme in Form einer Öl-in-Wasser-Emulsion

a)

| | |
|---|---|
| 5,0 g | Betainoctadecylester |
| 5,0 g | Glycerylstearat |
| 2,0 g | Cetylstearylalkohol, mit 12 Mol Ethylenoxid oxethyliert |
| 4,0 g | Cetylstearylalkohol, mit 6 Mol Ethylenoxid oxethyliert |
| 6,0 g | Cetylstearylalkohol |
| 6,0 g | Avocadoöl |
| 4,0 g | Mineralöl |
| 2,0 g | Dimethylpolysiloxan |

b)

| | |
|---|---|
| 4,0 g | Glycerin |
| 3,0 g | Glykolsäure |
| 2,0 g | Salicylsäure |
| 1,0 g | Zitronensäure |
| 1,0 g | Betain |
| 54,5 g | Wasser |

c)

| | |
|---|---|
| 0,5 g | Parfümöl |
| 100,0 g | |

Die Ölphase a) wird auf 75 Grad Celsius und unter Rühren in die auf 55 Grad Celsius erhitze Wasserphase b) emulgiert. Nach Abkühlung auf 45 Grad Celsius wird das Parfümöl zugesetzt und die Emulsion homogenisiert. Der pH-Wert wird mit Natriumhydroxid auf 4,0 eingestellt.

Die Hautcreme gemäß Beispiel 2 ist eine weiche Creme, die rasch in die Haut einzieht. Sie zeichnet sich durch ein

hervorragendes Hautgefühl und ausgezeichnete Verträglichkeit aus. Die Creme bewirkt nach mehrfacher Verwendung eine Reduzierung der Hautfältchen.

**Beispiel 3:** Hautcreme in Form einer Wasser-in-Öl-Emulsion

a)

| | |
|---|---|
| 4,00 g | Polyglyceryl-2-sesquiisostearat |
| 4,00 g | mit 7 Mol Ethylenoxid oxethyliertes hydriertes Rizinusöl |
| 8,00 g | Cetylstearylisononanoat |
| 8,00 g | Squalan |
| 2,00 g | Betainoctadecylester |
| 3,00 g | Bienenwachs |
| 2,00 g | Carnaubawachs |

b)

| | |
|---|---|
| 61,00 g | Wasser |
| 3,00 g | Glycerin |
| 2,00 g | Betaindodecylester |
| 0,50 g | Salicylsäure |
| 0,50 g | Glykolsäure |
| 0,50 g | Valin |

c)

| | |
|---|---|
| 0,50 g | Parfümöl |
| 1,00 g | Tocopherylacetat |
| 100,00 g | |

Die Ölphase a) wird auf 80 Grad Celsius aufgeschmolzen. In einem separaten Gefäß wird die Wasserphase b) auf 75 Grad Celsius erhitzt und unter Rühren der Ölphase zugesetzt. Nach Abkühlung auf 50 Grad Celsius werden das Parfümöl und das Tocopherylacetat eingearbeitet und die Emulsion intensiv homogenisiert.

Die Hautcreme gemäß Beispiel 3 ist eine weiche Creme, die sich auf der Haut sehr gut verteilen läßt und ein angenehmes, glattes Hautgefühl bewirkt. Zudem weist sie ausgezeichnete Hautschutzeigenschaften sowie eine hervorragende Verträglichkeit auf.

**Beispiel 4:** Öl-in-Wasser-Körperlotion

a)

| | |
|---|---|
| 10,00 g | Cetylstearyloctanoat |
| 3,00 g | Sorbitanmonostearat |
| 3,0 g | Mischung der Laurylester des Sorbitols und des Sorbitolanhydrids mit 20 Mol Ethylenoxid oxethyliert (CTFA-Bezeichnung Polysorbate-20) |
| 2,0 g | Dimethylpolysiloxan |
| 4,0 g | Cetylstearylalkohol |
| 2,0 g | Stearinsäure |
| 0,8 g | Betainoctadecylester |
| 0,1 g | para-Hydroxybenzoesäurepropylester |

b)

| | |
|---|---|
| 69,0 g | Wasser |
| 0,2 g | Betaindodecylester |
| 5,0 g | Glycerin |
| 0,3 g | Zitronensäure |
| 0,2 g | Salicylsäure |
| 0,1 g | Allantoin |

c)

| | |
|---|---|
| 0,3 g | Parfümöl |
| 100,0 g | |

Die Ölphase a) wird auf 75 Grad Celsius erhitzt und unter Rühren in die auf 70 Grad Celsius erhitzte Wasserphase b) einemulgiert. Nach Abkühlung auf 45 Grad Celsius wird das Parfümöl eingearbeitet und die Emulsion homogenisiert.

Die so hergestellte Körperlotion zieht rasch in die Haut ein, bewirkt ein glattes, angenehmes Hautgefühl und weist eine hervorragende Verträglichkeit auf.

**Beispiel 5:** Gesichts-Tonic

| | |
|---|---|
| 2,00 g | Betaindodecylester |
| 1,00 g | Zitronensäure |
| 4,00 g | Harnstoff |
| 2,00 g | Salicylsäure |
| 2,00 g | Milchsäure |
| 2,00 g | Glycerin |
| 2,00 g | Betain |
| 0,10 g | Hyaluronsäure |
| 69,90 g | Wasser |
| 15,00 g | Alkohol |
| 100,00 g | |

Das Mittel gemäß Beispiel 5 weist ausgezeichnete pflegende und antiseptische Eigenschaften auf und eignet sich ganz besonders zur Pflege unreiner und fettiger Haut.

Alle Prozentangaben stellen, soweit nicht anders angegeben, Gewichtsprozente dar.

**Patentansprüche**

1. Verwendung einer Zubereitung mit einem pH-Wert von 1 bis 7, enthaltend
0,1 bis 20 Gewichtsprozent mindestens eines Betainesters der allgemeinen Formel (I)

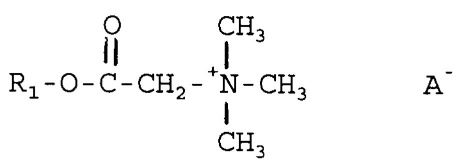

I,

wobei $R_1$ einen $C_{10}$- bis $C_{30}$-Alkylrest und $A^-$ ein Anion darstellt und
0,05 bis 20 Gewichtsprozent mindestens einer α-Hydroxysäure, welche ausgewählt ist aus Zitronensäure und Milchsäure,
zur Pflege der Haut.

2. Verwendung einer Zubereitung zur Pflege der Haut nach Anspruch 1, dadurch gekennzeichnet, daß die verwendete Zubereitung Betainoctadecylester und/oder Betaindodecylester enthält.

3. Hautpflegemittel mit einem pH-Wert von 1 bis 7 und einem Gehalt an einer Kombination aus

   a) 0,1 bis 20 Gewichtsprozent mindestens eines Betainesters der allgemeinen Formel (I)

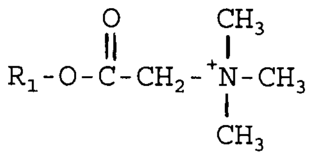 $A^-$ I,

wobei $R_1$ einen $C_{10}$- bis $C_{30}$-Alkylrest und $A^-$ ein Anion darstellt, und

b) 0,05 bis 20 Gewichtsprozent mindestens einer α-Hydroxysäure mit Ausnahme von Milchsäure und Zitronensäure.

**4.** Hautpflegemittel nach Anspruch 3, dadurch gekennzeichnet, daß es Betainoctadecylester und/oder Betaindodecylester enthält.

**5.** Hautpflegemittel nach einem der Ansprüche 3 bis 4, dadurch gekennzeichnet, daß es 0,5 bis 5 Gewichtsprozent der Komponente a) enthält.

**6.** Hautpflegemittel nach einem der Ansprüche 3 bis 5 dadurch gekennzeichnet, daß es 0,5 bis 6 Gewichtsprozent der Komponente b) enthält.

**7.** Hautpflegemittel nach einem der Ansprüche 3 bis 6, dadurch gekennzeichnet, daß die α-Hydroxysäure der Komponente b) ausgewählt ist aus Glykolsäure, Äpfelsäure, Weinsäure, Salicylsäure, Gluconsäure, Glucuronsäure und Zuckersäure.

Europäisches Patentamt

# EUROPÄISCHER RECHERCHENBERICHT

Nummer der Anmeldung
EP 96 10 1328

| EINSCHLÄGIGE DOKUMENTE | | | |
|---|---|---|---|
| Kategorie | Kennzeichnung des Dokuments mit Angabe, soweit erforderlich, der maßgeblichen Teile | Betrifft Anspruch | KLASSIFIKATION DER ANMELDUNG (Int.Cl.6) |
| A,D | DE-A-35 27 974 (WELLA)<br>* das ganze Dokument *<br>--- | 1-7 | A61K7/48<br>A61K7/50 |
| A | EP-A-0 413 528 (YU ET AL.)<br>* das ganze Dokument *<br>--- | 1-7 | |
| A | DE-A-19 50 643 (MANZKE)<br>* das ganze Dokument *<br>--- | 1-7 | |
| A | US-A-4 548 810 (ZOFCHAK)<br>* das ganze Dokument *<br>--- | 1-7 | |
| A | PATENT ABSTRACTS OF JAPAN<br>vol. 11, no. 222 (C-435) [2669]<br>& JP-A-62 039512 (YAKURIGAKU CHUO KENKYUSHO KK)<br>* Zusammenfassung *<br>----- | 1-7 | |
| | | | RECHERCHIERTE SACHGEBIETE (Int.Cl.6) |
| | | | A61K |

Der vorliegende Recherchenbericht wurde für alle Patentansprüche erstellt

| Recherchenort | Abschlußdatum der Recherche | Prüfer |
|---|---|---|
| DEN HAAG | 17.Oktober 1996 | Fischer, J.P. |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P04C03)